# EUROPÄISCHE PATENTSCHRIFT

(11) **EP 1 116 733 B1**
(45) Veröffentlichungstag und Bekanntmachung des Hinweises auf die Patenterteilung: **23.04.2003**
(21) Anmeldenummer: 00710047.2
(22) Anmeldetag: 29.12.2000
(51) Int. Cl.: C08F 226/06, C08F 226/02, C08F 220/58, A61K 7/00, A61K 9/00

(54) **Wasserlösliche oder wasserquellbare vernetzte Copolymere**
Water-soluble or water-swellable crosslinked copolymers
Copolymères réticulés solubles ou gonflables dans l'eau:

(30) Priorität: 11.01.2000 DE 10000648
(43) Veröffentlichungstag der Anmeldung: 18.07.2001
(73) Patentinhaber: Clariant GmbH, 65929 Frankfurt am Main (DE)
(72) Erfinder: Löffler, Matthias Dr., 65527 Niedernhausen (DE); Morschhäuser, Roman Dr., 55122 Mainz (DE)

(56) Entgegenhaltungen:
- EP-A- 0 483 638
- EP-A- 0 815 828
- US-A- 4 619 773

## Beschreibung

Die vorliegende Erfindung betrifft wasserlösliche oder wasserquellbare Copolymerisate auf Basis von Ammoniumsalzen von Acrylamido-alkylsulfonsäuren und cyclischen N-Vinylcarbonsäureamiden bzw. cyclischen und linearen N-Vinylcarbonsäureamiden, deren Herstellung und deren Verwendung als Verdicker, Stabilisator von Emulsionen und Dispersionen und als Gleitmittel in kosmetischen und pharmazeutischen Mitteln.

Wasser- oder lösungsmittelhaltige Mehrkomponentensysteme wie Lösungen, Emulsionen oder Suspensionen werden häufig aus ökonomischen oder anwendungstechnischen Gründen oder aus Stabilitätsgründen auf höhere Viskositäten eingestellt bzw. verdickt.
So kann z.B. durch Erhöhung der Viskosität der externen oder internen Phase von Emulsionen oder Suspensionen erreicht werden, dass die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert. Dies gilt insbesondere für Hautpflegemittel und pharmazeutische Salben auf der Haut. Bei vielen technischen Produkten wie Tapetenablösem, Abbeizmitteln oder Flugzeugenteisern verhindert die erhöhte Viskosität ein vorzeitiges Abfließen von der zu behandelnden Fläche. Durch die gleichmäßigere Verteilung und verlängerte Einwirkdauer wird so die Wirksamkeit erhöht. Neben den erwähnten anwendungstechnischen Vorteilen bietet die hohe Viskosität solcher Präparate auch weitere Vorteile bei der Herstellung, Verpackung, Abfüllung und Lagerung sowie beim Transport, insbesondere ist hier aus sicherheitstechnischer Hinsicht die Verdickung saurer Medien von Bedeutung. Generell sind die rheologischen Eigenschaften bei der Herstellung und/oder Formulierung kosmetischer, pharmazeutischer oder technischer Präparate ein entscheidendes Kriterium für den Einsatz dieser Produkte in der Praxis. Die eingesetzten Verdicker sollen dabei bereits in möglichst geringen Einsatzmengen zu einer ausreichenden Verdickung führen. Jedoch sollen die Farbe und prinzipiellen Eigenschaften des zu verdickenden Mediums nicht verändert werden.

Um die rheologischen Eigenschaften von wässrigen oder lösungsmittelhaltigen Systemen, Emulsionen, Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyacrylsäure und verschiedene Salze der Polyacrylsäure, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen.

Die genannten Verbindungen zeigen jedoch bei der Anwendung vielfältige Nachteile. So sind z.B. die Cellulosederivate bzw. allgemein die auf natürlichen Rohstoffen basierenden Materialien und die daraus resultierenden Formulierungen sehr anfällig gegen Bakterien. Anwendungstechnisch fallen sie zumeist durch die Bildung unangenehmer, "fadenziehender" Gele auf. Fettsäurepolyethylenglykolester neigen in Gegenwart von Wasser zur Hydrolyse, die dabei entstehenden unlöslichen Fettsäuren verursachen unerwünschte Trübungen. Verdickungsmittel natürlichen Ursprungs (z.B. Agar-Agar oder Traganth) weisen je nach Herkunft stark schwankende Zusammensetzung auf.

In EP-A-0 816 403 und WO 98/00094 sind vernetzte Homopolymere aus
2-Acrylamldo-2-methyl-propan-sulfonaten und deren Verwendung als Verdicker beschrieben. EP-A-0 510 246 beschreibt vernetzte Copolymere aus
N-Vinylcarbonsäureamiden und ungesättigten, mit einer Sulfonatgruppe substituierten Alkylamiden, die ebenfalls als Verdicker geeignet sind.
In US 5 080 809 sind unvernetzte Copolymerisate aus N-Vinylpyrrolidon und 2-Acrylamido-2-methyl-propan-sulfonat beschrieben. DE 199 05 639.0 beschreibt vernetzte Polymerisate aus nichtcyclischen N-Vinylcarbonsäureamiden und Acrylamidoalkylsulfonsäuren.

Überraschenderweise wurde nun gefunden, dass Alkali-, Erdalkali- oder Ammoniumsalze verschiedener Acrylamidoalkylsulfonsäuren, in für kosmetische Anwendungen unbedenklichen Lösungsmitteln, wie Alkoholen oder Alkoholgemischen, hinreichend löslich sind und somit für eine Copolymerisation mit ebenfalls in diesen Lösungsmitteln löslichen cyclischen N-Vinylcarbonsäureamiden bzw. Mischungen aus mehreren zyklischen N-Vinylcarbonsäureamiden oder Mischungen aus zyklischen und linearen N-Vinylcarbonsäureamiden, gegebenenfalls mit weiteren Monomeren, sowie als Vernetzer wirkenden Monomeren hervorragend geeignet sind. Im Gegensatz hierzu muss gemäß dem Stand der Technik in einem aprotischen Lösungsmittel gearbeitet werden. Da das für die Polymerisation bevorzugt eingesetzte Ammoniumsalz der 2-Acrylamido-2-methyl-propan-sulfonsäure in ionischer Form vorliegt, braucht das erhaltene vernetzte Copolymer nicht mehr umständlich nachträglich neutralisiert zu werden, sondern ist sofort nach Polymerisation und Abtrennung des Lösungsmittels als Verdickungsmittel einsetzbar. Als weiterer Vorteil lässt sich durch geeignete Wahl des (oder der) Comonomeren (cyclische N-Vinylcarbonsäureamide, Mischungen aus cyclischen und linearen N-Vinylcarbonsäureamiden) das Verhältnis von ionischen zu neutralen Bausteinen steuern und damit die Verdickungswirkung und Salzstabilität regulieren und speziellen Anforderungen besser anpassen. Durch die Polymerisation in Alkohol bzw. Alkoholgemischen mit einem Wassergehalt von kleiner als 10 Gew.-% und hier insbesondere in tert.-Butanol erhält man außerdem Produkte, die bezüglich ihres Restgehaltes an im Produkt verbleibendem Lösungsmittel toxikologisch unbedenklich sind und so zum Beispiel in kosmetischen Produkten Verwendung finden können.

Gegenstand der Erfindung sind vernetzte Copolymere bestehend im wesentlichen aus
a1) 1 bis 50 Gew.-% der wiederkehrenden Struktureinheit der Formel (1) 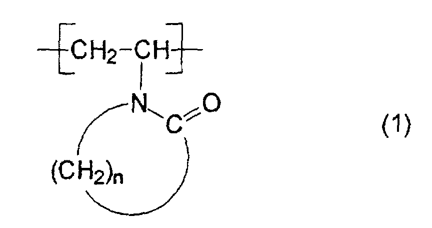 wobei n eine ganze Zahl von 2 bis 9 bedeutet,
   oder
a2) 1 bis 50 Gew.-% einer Mischung der wiederkehrenden Struktureinheit der Formel (1) und der wiederkehrenden Struktureinheit der Formel (2) 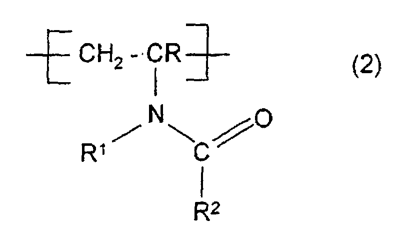 wobei R, R¹ und R² gleich oder verschieden sein können und Wasserstoff oder eine lineare oder verzweigte Alkyl- oder Alkenylgruppe mit jeweils 1 bis 30, bevorzugt 1 bis 20, insbesondere 1 bis 12 C-Atomen bedeuten und
b) 49,99 bis 98,99 Gew.-% der wiederkehrenden Struktureinheit der Formel (3) 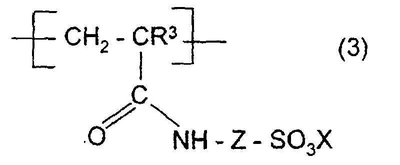 worin R³ Wasserstoff, Methyl oder Ethyl, Z C₁-C₈-Alkylen, n eine ganze Zahl von 2 bis 9 und X ein Alkali-, Erdalkali- oder Ammonium-Ion bedeutet, sowie
c) 0,01 bis 8, vorzugsweise 0,01 bis 5 Gew.-% vernetzenden Strukturen, die aus Monomeren mit mindestens zwei olefinischen Doppelbindungen hervorgegangen sind.

Bevorzugte erfindungsgemäße Copolymere enthalten 2 bis 30, insbesondere 3 bis 15 Gew.-% an Struktureinheiten der allgemeinen Formel (1), bzw. (1) und (2), vorzugsweise abgeleitet von N-Vinylpyrrolidon, 69,5 bis 97,5, insbesondere 84,5 bis 96,5 Gew.-% an Struktureinheiten der allgemeinen Formel (3), vorzugsweise abgeleitet vom Ammoniumsalz der 2-Acrylamido-2-methyl-propan-sulfonsäure und 0,2 bis 3, insbesondere 0,5 bis 2 Gew.-% an vernetzenden Strukturen, die aus Monomeren mit mindestens zwei olefinischen Doppelbindungen hervorgegangen sind. Das Mischungsverhältnis der den Struktureinheiten 1 und 2 zugrunde liegenden Monomeren kann innerhalb beliebiger Grenzen variieren.

Vernetzende Strukturen, die aus Monomeren mit mindestens zwei olefinischen Doppelbindungen hervorgegangen sind, leiten sich vorzugsweise ab von Acryl- oder Methacrylsäureallylester, Dipropylenglykol-diallylether, Polyglykoldiallylether, Triethylen-glykoldivinylether, Hydrochinon-diallylether, Tetraallyloxyethan oder anderen Allyl- oder Vinylethem multifunktioneller Alkohole, Tetraethylenglykoldiacrylat, Triallylamin, Trimethylolpropandiallylether, Methylen-bis-acrylamid oder Divinylbenzol.
Besonders bevorzugt leiten sich die vemetzenden Strukturen ab von Monomeren der allgemeinen Formel (4), 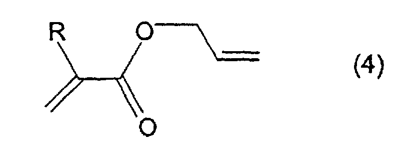 worin R Wasserstoff, Methyl oder Ethyl bedeutet.

Die Herstellung der erfindungsgemäßen Copolymeren erfolgt in der Weise, dass man die den wiederkehrenden Struktureinheiten der Formeln (1), (2) und (3) entsprechenden Monomeren in einem protischen Lösungsmittel löst oder dispergiert, zu dieser Lösung oder Dispersion einen oder mehrere Vemetzer mit mindestens zwei olefinischen Doppelbindungen zugibt und die Polymerisation in an sich bekannter Weise durch Zugabe einer radikalbildenden Verbindung startet.

Bevorzugt wird das Ammoniumsalz der Acrylamidopropansulfonsäure einpolymerisiert. Anstelle dieses Ammoniumsalzes kann man auch die freie Acrylamidopropansulfonsäure einsetzen und vor der Zugabe der restlichen Monomere aus der freien Säure durch Einleiten von Ammoniak das Ammoniumsalz erzeugen.

Die Polymerisationsreaktion erfolgt vorzugsweise in einem wasserlöslichen Alkohol oder einem Gemisch mehrerer Alkohole mit 1 bis 6 C-Atomen, vorzugsweise in tert.-Butanol. Der Wassergehalt des Alkohols oder des Gemisches mehrerer Alkohole darf 10 Gew.-% nicht überschreiten, da sonst im Verlauf der Polymerisation Klumpenbildung auftreten kann. Konkret hat die Wahl der Art und der Menge des Lösungsmittels so zu erfolgen, dass das Salz der Acrylamidoalkylsulfonsäure der Formel 1, insbesondere der 2-Acrylamido-2-methyl-propan-sulfonsäure darin weitgehend löslich oder dispergierbar ist. Unter weitgehend löslich oder dispergierbar ist zu verstehen, dass sich auch nach Abstellen des Rührwerks kein festes Material aus der Lösung oder Dispersion absetzt. Das im Verlaufe der Reaktion entstehende Polymerisat soll hingegen in dem gewählten Lösungsmittel (oder -gemisch) weitgehend unlöslich sein. Unter weitgehend unlöslich ist hierbei zu verstehen, dass im Verlauf der Polymerisation eine gut rührbare, breiige Polymerpaste entsteht, in der sich keine Klumpen oder Verklebungen bilden dürfen. Das durch Absaugen der Paste erhältliche Filtrat darf einen Feststoffgehalt von maximal 5 Gew.-% aufweisen. Sind die Copolymere in stärkerem Ausmaß im gewählten Lösungsmittel oder Lösungsmittelgemisch löslich, kann es beim Trocknen der Polymerisatpaste zu Verklumpungen kommen.
Die Polymerisationsreaktion selbst wird in an sich bekannter Weise durch radikalbildende Verbindungen wie Azoinitiatoren (z.B. Azo-bis-isobutyronitril), Peroxiden (z.B. Dilaurylperoxid) oder Persulfaten in einem geeigneten Temperaturintervall von 20 bis 120°C, vorzugsweise zwischen 40 und 80°C, ausgelöst und über einen Zeitraum von 30 min. bis mehreren Stunden fortgeführt.
Die Copolymerzusammensetzung lässt sich durch Variation des oben beschriebenen Einsatzverhältnisses der Monomere sowie des Anteils an Vemetzer variieren und so zur Erstellung eines maßgeschneiderten Eigenschaftsprofils verwenden. Durch verstärkten Einbau von Ammoniumsalzen der Acrylamidosulfonsäuren kann beispielsweise die verdickende Wirkung der Polymerisate verbessert werden, während durch Einbau von mehr cyclischem N-Vinylcarbonsäureamid die Elektrolytverträglichkeit der Polymerisate und deren Löslichkeit in nicht wässrigen Systemen verbessert werden kann.
Im Gegensatz zu den Polymerisaten auf Basis Acrylsäure, die im neutralen oder leicht alkalischen Bereich in 1 %iger wässriger Lösung durchaus Viskositäten von mehr als 30 000 mPa·s zeigen, deren Verdickungsvermögen (bzw. die gemessene Viskosität) jedoch mit sinkendem pH-Wert stark nachlässt, können die erfindungsgemäß beschriebenen Copolymere ihre Viskosität bis zu einem sauren pH-Wert von ca. 3 aufrecht erhalten.

### Beispiel 1

In einem 1000 ml Kolben mit Ankerrührer, Rückflusskühler, Innenthermometer, Einleitungsmöglichkeit für N₂ und NH₃ wurden 490,5 g tert. Butanol und 11,5 g Wasser vorgelegt. Anschließend wurden 80,75 g 2-Acrylamido-2-methyl-propansulfonsäure eingetragen und unter starkem Rühren dispergiert, wobei eine Trübung des Lösungsmittels erhalten blieb. Über einen Zeitraum von 30 min. leitete man 6,64 g Ammoniak in den überstehenden Gasraum ein und rührte mindestens weitere 30 min. nach bis sich ein pH-Wert von 6-7 eingestellt hatte. Man gab 4,10 g N-Vinylpyrrolidon und 0,8 g Methacrylsäureallylester hinzu und spülte die Vorlage jeweils mit tert. Butanol (ca. 6 ml) nach, um Verluste bei der Zugabe zu minimieren. Das Reaktionsgemisch wurde dann auf eine Temperatur von T = 60°C erwärmt, wobei die Reaktionsmischung durch gleichzeitiges Einleiten von N2 inertisiert wurde. Nach Erreichen der Temperatur von T = 60°C wurden 1,0 g Dilaurylperoxid zugegeben. Die Reaktion sprang unmittelbar nach Zugabe des Initiators an, was an einem Anstieg der Temperatur und am Ausflocken des Polymers zu erkennen war. Etwa 15 min. nach dem Einsetzen der Polymerisationsreaktion wurde die Stickstoffzufuhr abgestellt. Ungefähr 30 min. nach Zugabe des Starters Dilaurylperoxid erreichte die Temperatur ein Maximum (ca. 65 - 70°C). Weitere 30 min. nach Durchlaufen dieses Maximums wurde zum Rückfluss erhitzt und unter diesen Bedingungen zwei Stunden nachgerührt. Der Inhalt des Reaktionsgefäßes nahm im Verlauf der Reaktion eine breiartige Konsistenz an, war aber noch gut rührbar. Anschließend wurde auf Raumtemperatur abgekühlt und der Feststoff abgesaugt. Die Paste wurde bei 60 - 70°C über 24 Stunden im Vakuumtrockenschrank getrocknet. Man erhielt 92,2 g eines feinen weißen Pulvers.

### Beispiel 2

Das Beispiel 1 wurde wiederholt mit dem Unterschied, dass anstelle von Methacrylsäureallylester als Vernetzer 1,65 g Trimethylolpropanmethacrylat verwendet wurden.

### Beispiel 3

Entsprechend Beispiel 1 wurde das vernetzte Copolymer aus 35 g 2-Acrylamido-2-methyl-propan-sulfonsäure, 55 g N-Vinylpyrrolidon und 1,9 g Trimethylolpropan-triacrylat hergestellt.

### Beispiel 4

Entsprechend Beispiel 1 wurde das vernetzte Copolymer aus 77,5 g 2-Acrylamido-2-methyl-propan-sulfonsäure, 8,9 g N-Vinylpyrrolidon, 4,2 g N-Vinylformamid und 1,8 g Trimethylolpropan-triacrylat hergestellt.

### Vergleichsbeispiel 1

Entsprechend Beispiel 1 wurde ein vemetztes Homopolymer aus 85 g 2-Acrylamido-2-methyl-propan-sulfonsäure und 0,8 g Methacrylsäureallylester hergestellt.

### Testergebnisse:

Die gemäß den Beispielen gewonnenen Pulver wurden jeweils zu 1,0 Gew.-% in destilliertem Wasser aufgelöst und die Viskosität der dabei gebildeten Gele bei 25°C gemessen. Hierzu wurden in einem 600 ml Becherglas jeweils 5 g getrocknetes Polymer-Pulver in 495 g destilliertem Wasser eingerührt und die Viskosität des dabei gebildeten Gels mit einem Brookfield Viskosimeter Typ RVT bei 20 Upm gemessen. Die so hergestellten Gele eignen sich insbesondere für kosmetische Anwendungen, da sie beim Verteilen auf dem Körper ein angenehmes Hautgefühl hervorrufen.

Die Bestimmung der Säurestabilität erfolgte ebenfalls durch Viskositätsmessung mit dem Brookfield-Viskosimeter. Hierzu wurde das nach Herstellungsbeispiel 1 hergestellte Copolymer mit einem handelsüblichen Polymer auf Basis Acrylsäure (Carbopol® 934 der Fa. Goodrich) verglichen. Von beiden Polymeren wurden nach der oben beschriebenen Methode 1,0 %ige Gele hergestellt, deren pH-Wert gegebenenfalls durch Zugabe von NaOH bzw. H₃PO₄ auf einem sauren (pH = ca. 3) und einen neutralen (pH = 6-7) eingestellt wurde.

**Tabelle:**

| gemessene Viskositäten der 1,0 %igen Gele | | |
|---|---|---|
| pH-Wert | Polymer Beispiel 1 | Carbopol 934 |
| 6 - 7 | 65 600 mPa·s | 76 600 mPa·s |
| ca. 3 | 52 100 mPa·s | 140 mPa·s |

Wie der Tabelle zu entnehmen ist, zeigen im Gegensatz zu den auf Basis von Acrylsäure aufgebauten Polymere die erfindungsgemäß beschriebenen Polymere auch bei sauren pH-Werten sehr gute Verdickungseigenschaften.

Die erfindungsgemäßen Copolymere zeichnen sich aus durch ihre stark verdickende Wirkung, insbesondere in kosmetischen und pharmazeutischen Zubereitungen bei Konzentrationen an festem Copolymer von 0,1 bis 5 Gew.-%, bevorzugt von 2 bis 0,5 Gew.-%, besonders bevorzugt von 0,7 bis 1 Gew.-%, bezogen auf das fertige Mittel. Bei Raumtemperatur in deionisiertem Wasser werden bei einem pH-Wert von 6 bis 7 Viskositäten von mehr als 60 000 mPa·s erreicht.

Die erfindungsgemäßen Copolymeren zeigen in einem weiten pH-Bereich, insbesondere im Bereich von pH 2,5 bis 7 nur relativ geringe Viskositätsänderungen. Außerdem behalten sie in den Formulierungen ihre gute Wasserlöslichkeit bei und können leicht von der Haut abgewaschen werden. Ihre verdickenden und stabilisierenden Eigenschaften kommen auch in wässrig, alkoholischen und/oder glykolhaltigen Lösungen zur Wirkung. Sie sind UV-stabil und in einem weiten Temperaturbereich von 0 bis 50°C stabil.

Durch Variation der Monomeren Acrylamidosulfonsäuresalz und N-Vinylcarbonsäureamid, sowie des Anteils an Vernetzer werden Copolymere erhalten, die sowohl in Öl-in-Wasser Emulsionen, als auch in Wasser-in-Öl Emulsionen bei einem pH von 7 bis 2,5 als Verdicker eingesetzt werden können. Abhängig davon, ob Lotionen mit einer vergleichsweise niedrigen oder Cremes und Salben mit hohen Viskositäten hergestellt werden sollen, enthalten Emulsionen einen Ölkörper, bestehend im wesentlichen aus Emulgatorlen und einer Ölphase in den Gewichtsmengen von 5 bis 95 %, vorzugsweise 25 bis 85 % und der an 100 Gew.-% fehlenden Menge Wasser. Als Ölkörper kommen pflanzliche, tierische, mineralische und synthetische Öle zum Einsatz, beispielsweise Guerbetalkohole mit 6 bis 18, vorzugsweise 8 bis 10 Kohlenstoffatomen, Ester von linearen C₆-C₁₃-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe.

Die Emulsionen können als Hautpflegemittel, wie beispielsweise Tagescremes, Nachtcremes, Pflegecremes, Nährcreme, Bodylotions, Salben und dergleichen vorliegen und als weitere Hilfs- und Zusatzstoffe, Co-Emulgatoren, Überfettungsmittel, Fette, Wachse, Stabilisatoren, biogene Wirkstoffe, Glycerin, Konservierungsmittel, Perlglanzmittel, Farb- und Duftstoffe enthalten.

Als Überfettungsmittel können Substanzen wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure.
Als Perlglanzmittel kommen beispielsweise Glycoldistearinsäureester wie Ethylenglycoldistearat, aber auch Fettsäuremonoglykolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106, zusammengestellt sind.

Der Gesamtanteil der Hilfs- und Zusatzstoffe kann 1 bis 10, vorzugsweise 2 bis 5 Gew.-%, bezogen auf das Mittel, betragen.
Die Herstellung der Mittel kann in an sich bekannter Weise, d.h. beispielsweise durch Heiß-, Heiß-Heiß/Kalt- bzw. PIT-Emulgierung erfolgen.

Die nachfolgenden Beispiele sollen die Anwendungsmöglichkeiten der erfindungsgemäßen Verdicker näher erläutern, ohne diese darauf einzuschränken. Die Prozentangaben sind in allen Fällen Gewichtsprozente.

### Beispiel 1: O/W-Creme

| | | |
|---|---|---|
| A | Hostacerin DGI | 2,00 % |
| | Mineralöl, niedrigviskos | 8,00 % |
| | Isopropylpalmitat | 4,00 % |
| | Eutanol G | 4,00 % |
| | | |
| B | Copolymer 1 | 1,20 % |
| | | |
| C | Hostapon KCG | 0,80 % |
| | Wasser | ad 100 % |
| | Konservierungsmittel | q.s. |
| D | Duftstoffe | 0,40 % |

### Herstellweise

- I: Einrühren von B in A, dann Hinzufügen von C und gut Verrühren
- II: Einrühren von D in I
- III: Homogenisieren

### Beispiel 2: O/W-Hautmilch

| | | |
|---|---|---|
| A | Hostacerin DGMS | 2,00 % |
| | Mineralöl, hochviskos | 8,00 % |
| | Isopropylpalmitat | 5,00 % |
| | Cetiol 868 | 4,00 % |
| | | |
| B | Copolymer 2 | 0,50 % |
| | | |
| C | Hostapon KCG | 2,00 % |
| | Glycerin | 4,00 % |
| | Wasser | ad 100 % |
| | Konservierungsmittel | q.s. |
| | | |
| D | Duftstoffe | 0,30 % |

### Herstellweise:

- I: Aufschmelzen von A auf ca. 70°C; Zugabe von B
- II: Erwärmen von C auf ca. 70°C
- III: Einrühren von II in I bis zum Erkalten rühren
- IV: Zugabe von D bei ca. 35°C
- V: Homogenisieren

### Beispiel 3: O/W-Hautmilch

| | | |
|---|---|---|
| A | Hostacerin DGL | 2,00 % |
| | Isopropylpalmitat | 4,00 % |
| | Mandelöl | 5,00 % |
| | Weizenkeimöl | 1,00 % |
| | Cetiol SN | 8,00 % |
| | | |
| B | Copolymer 1 | 0,60 % |
| | | |
| C | Wasser | ad 100 % |
| | Konservierungsmittel | q.s. |
| | | |
| D | Duftstoffe | 0,30 % |

### Herstellweise:

- I: A und B mischen und in C einrühren
- II: D hinzufügen
- III: homogenisieren

### Beispiel 4: O/W-Hautmilch

| | | |
|---|---|---|
| A | Hostaphat CG 120 | 1,50 % |
| | Mineralöl, niedrigviskos | 5,00 % |
| | Miglyol 812 | 4,00 % |
| | Isopropylpalmitat | 6,00 % |
| | Sojaöl | 3,00 % |
| | Jojobaöl | 2,00 % |
| | | |
| B | Copolymer 1 | 0,80 % |
| | | |
| C | Hostapon KCG | 1,00 % |
| | Wasser | 100 % |
| | Glycerin | 3,00 % |
| | Soda (10 % in Wasser) | 1,20 % |
| | Konservierungsmittel | q.s. |
| | | |
| D | Duftstoffe | 0,30 % |

### Herstellweise:

- I: B in A einrühren, C dazugeben und gut vermischen
- II: D hinzufügen
- III: homogenisieren

| Handelsprodukte | | |
|---|---|---|
| ®Hostacerin DGI | (Clariant GmbH) | Polyglyceryl-2-Sesquiisostearat |
| ®Eutanol G | (Henkel KGaA) | Octyldodecanol |
| Copolymer 1 | | Copolymer gemäß Beispiel 1 |
| Copolymer 2 | | Copolymer gemäß Beispiel 2 |
| ®Hostapon KCG | (Clariant GmbH) | Natriumcocoylglutamat |
| Hostacerin DGMS | (Clariant GmbH) | Polyglyceryl-2-stearat |
| ®Cetiol 868 | (Henkel KGaA) | Octylstearat |
| Hostacerin DGL | (Clariant GmbH) | Polyglyceryl-2 PEG-10 Laurat |
| ®Cetiol SN | (Henkel KGaA) | Cetearylisononat |
| ®Hostaphat CG 120 | (Clariant GmbH) | Octyldecylphosphat |
| ®Miglyol 812 | (Dynamit Nobel AG) | Capryltriglycerid |

## Patentansprüche

1. Wasserlösliche oder wasserquellbare vernetzte Copolymere bestehend im wesentlichen aus
a1) 1 bis 50 Gew.-% der wiederkehrenden Struktureinheit der Formel (1) 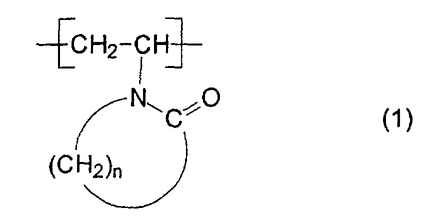 wobei n eine ganze Zahl von 2 bis 9 bedeutet,
oder
a2) 1 bis 50 Gew.-% einer Mischung der wiederkehrenden Struktureinheit der Formel (1) und der wiederkehrenden Struktureinheit der Formel (2) 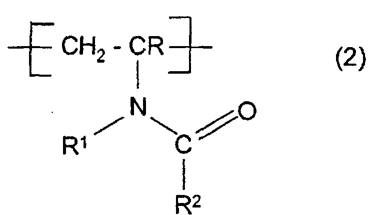 wobei R, R¹ und R² gleich oder verschieden sein können und Wasserstoff oder eine lineare oder verzweigte Alkyl- oder Alkenylgruppe mit jeweils 1 bis 30, bevorzugt 1 bis 20, insbesondere 1 bis 12 C-Atomen bedeuten und
b) 49,99 bis 98,99 Gew.-% der wiederkehrenden Struktureinheit der Formel (3) 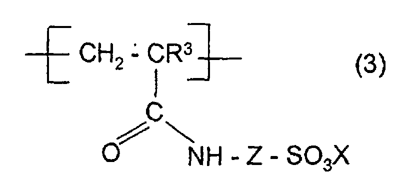 worin R³ Wasserstoff, Methyl oder Ethyl, Z C₁-C₈-Alkylen, n eine ganze Zahl von 2 bis 9 und X ein Alkali-, Erdalkali- oder Ammonium-Ion bedeutet, sowie
c) 0,01 bis 8 Gew.-% vernetzenden Strukturen, die aus Monomeren mit mindestens zwei olefinischen Doppelbindungen hervorgegangen sind.

2. Copolymere nach Anspruch 1, bestehend im wesentlichen aus 2 bis 30 Gew.-% der wiederkehrenden Struktureinheit der Formel 1 oder einer Mischung der wiederkehrenden Struktureinheiten der Formeln 1 und 2, 69,5 bis 97,5 Gew.-% der wiederkehrenden Struktureinheit der Formel 3 und 0,2 bis 3 Gew.-% vernetzender Strukturen, die aus Monomeren mit mindestens zwei olefinischen Doppelbindungen hervorgegangen sind.

3. Copolymere nach Anspruch 1, bestehend im wesentlichen aus 3 bis 15 Gew.-% der wiederkehrenden Struktureinheit der Formel 1 oder einer Mischung der wiederkehrenden Struktureinheiten der Formeln 1 und 2, 84,5 bis 96,5 Gew.-% der wiederkehrenden Struktureinheit der Formel 3 und 0,5 bis 2 Gew.-% vernetzender Strukturen, die aus Monomeren mit mindestens zwei olefinischen Doppelbindungen hervorgegangen sind.

4. Copolymere nach Anspruch 1, enthaltend vernetzende Strukturen, die aus Allyl(meth)acrylat hervorgegangen sind.

5. Verfahren zur Herstellung der wasserlöslichen oder wasserquellbaren Copolymere nach Anspruch 1, **dadurch gekennzeichnet, dass** man die den wiederkehrenden Struktureinheiten der Formeln 1, 2 und 3 entsprechenden Monomeren in einem protischen Lösungsmittel löst oder dispergiert, zu dieser Lösung oder Dispersion einen oder mehrere Vernetzer mit mindestens zwei olefinischen Doppelbindungen zugibt und die Polymerisation durch Zugabe einer radikalbildenden Verbindung startet.

6. Verwendung der wasserlöslichen oder wasserquellbaren Copolymere nach Anspruch 1 als Verdicker.

7. Kosmetische oder pharmazeutische Zubereitungen enthaltend ein wasserlösliches oder wasserquellbares Copolymer nach Anspruch 1.

## Claims

1. A water-soluble or water-swellable crosslinked copolymer consisting essentially of
a1) 1 to 50% by weight of the repeating structural unit of the formula (1) 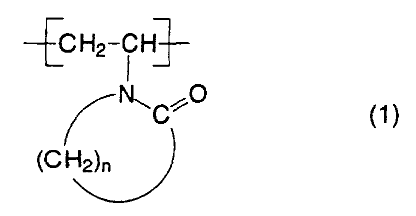 where n is an integer from 2 to 9,
or
a2) 1 to 50% by weight of a mixture of the repeating structural unit of the formula (1) and of the repeating structural unit of the formula (2) 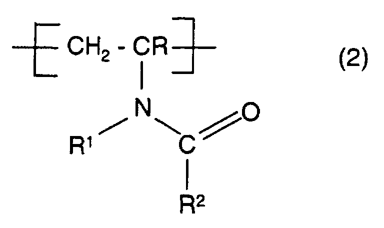 where R, R¹ and R² may be identical or different and are hydrogen or a linear or branched alkyl or alkenyl group having in each case 1 to 30, preferably 1 to 20, in particular 1 to 12, carbon atoms and
b) 49.99 to 98.99% by weight of the repeating structural unit of the formula (3) 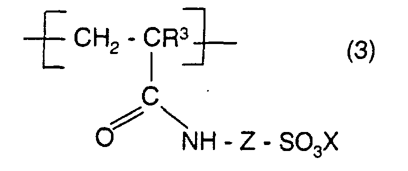 in which R³ is hydrogen, methyl or ethyl, Z is C₁-C₈-alkylene, n is an integer from 2 to 9, and X is an alkali metal, alkaline earth metal or ammonium ion, and
c) 0.01 to 8% by weight of crosslinking structures resulting from monomers having at least two olefinic double bonds.

2. The copolymer as claimed in claim 1, consisting essentially of 2 to 30% by weight of the repeating structural unit of the formula 1 or a mixture of the repeating structural units of the formulae 1 and 2, 69.5 to 97.5% by weight of the repeating structural unit of the formula 3 and 0.2 to 3% by weight of crosslinking structures resulting from monomers having at least two olefinic double bonds.

3. The copolymer as claimed in claim 1, consisting essentially of 3 to 15% by weight of the repeating structural unit of the formula 1 or a mixture of the repeating structural units of the formulae 1 and 2, 84.5 to 96.5% by weight of the repeating structural unit of the formula 3 and 0.5 to 2% by weight of crosslinking structures resulting from monomers having at least two olefinic double bonds.

4. The copolymer as claimed in claim 1, comprising crosslinking structures resulting from allyl (meth)acrylate.

5. A process for the preparation of the water-soluble or water-swellable copolymers as claimed in claim 1, which comprises dissolving or dispersing the monomers corresponding to the repeating structural units of the formulae 1, 2 and 3 in a protic solvent, adding one or more crosslinkers having at least two olefinic double bonds to this solution or dispersion, and starting the polymerization by adding a free-radical-forming compound.

6. The use of the water-soluble or water-swellable copolymers as claimed in claim 1 as thickeners.

7. A cosmetic or pharmaceutical preparation comprising a water-soluble or water-swellable copolymer as claimed in claim 1.

## Revendications

1. Copolymères réticulés hydrosolubles ou gonflables dans l'eau constitués essentiellement par
a1) 1 à 50 % en poids de l'unité de structure récurrente de formule (1) 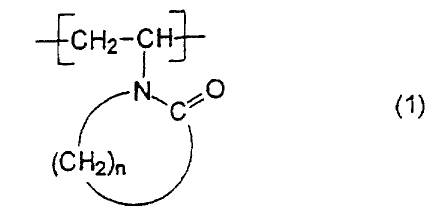 n étant un entier de 2 à 9,
ou
a2) 1 à 50 % en poids d'un mélange de l'unité de structure récurrente de formule (1) et de l'unité de structure récurrente de formule (2) 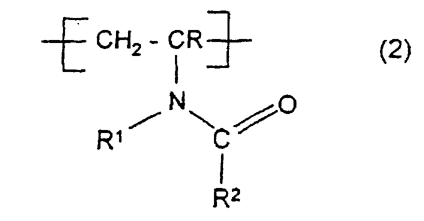 où R, R¹ et R² peuvent être identiques ou différents et représentent un atome d'hydrogène
ou un groupe alkyle ou alcényle linéaire ou ramifié chacun ayant 1 à 30, de préférence 1 à 20, en particulier 1 à 12 atomes de carbone et
b) 49,99 à 98,99 % en poids de l'unité de structure récurrente de formule (3) 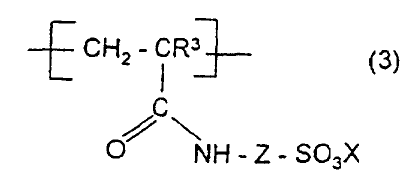 où R³ représente un atome d'hydrogène, un groupe méthyle ou éthyle, Z représente un groupe alkylène en C₁-C₈, n est un entier de 2 à 9 et X représente un ion alcalin, alcalino-terreux ou ammonium, ainsi que
c) 0,01 à 8 % en poids de structures réticulantes qui proviennent de monomères présentant au moins deux doubles liaisons oléfiniques.

2. Copolymères selon la revendication 1, constitués essentiellement par 2 à 30 % en poids de l'unité de structure récurrente de formule 1 ou un mélange des unités de structure récurrentes de formules 1 et 2, par 69,5 à 97,5 % en poids de l'unité de structure récurrente de formule 3 et par 0,2 à 3 % en poids de structures réticulantes, qui proviennent de monomères présentant au moins deux doubles liaisons oléfiniques.

3. Copolymères selon la revendication 1, constitués essentiellement par 3 à 15 % en poids de l'unité de structure récurrente de formule 1 ou d'un mélange des unités de structure récurrentes de formules 1 et 2, par 84,5 à 96,5 % en poids des unités de structure récurrentes de formule 3 et par 0,5 à 2 % en poids de structures réticulantes, qui proviennent de monomères présentant au moins deux doubles liaisons oléfiniques.

4. Copolymères selon la revendication 1, contenant des structures réticulantes qui proviennent du (méth)acrylate d'allyle.

5. Procédé pour la préparation des copolymères hydrosolubles ou gonflables dans l'eau selon la revendication 1, **caractérisé en ce qu'**on dissout ou disperse dans un solvant organique protique les monomères correspondants aux unités de structure récurrentes de formules 1, 2 et 3, on ajoute à cette solution ou dispersion un ou plusieurs réticulants avec au moins deux doubles liaisons oléfiniques et on amorce la polymérisation par ajout d'un composé radicalaire.

6. Utilisation des copolymères hydrosolubles ou gonflables dans l'eau selon la revendication 1 en tant qu'épaississant.

7. Préparations cosmétiques ou pharmaceutiques renfermant un copolymère hydrosoluble ou gonflable dans l'eau selon la revendication 1.
